# EUROPEAN PATENT SPECIFICATION

(11) **EP 2 036 924 B1**
(45) Date of publication and mention of the grant of the patent: **30.11.2011**
(21) Application number: 08171373.7
(22) Date of filing: 04.10.2002
(51) Int. Cl.: C07K 14/59, C07K 5/083, C07K 5/103, C07K 7/06, C07K 7/08, A61K 38/00

(54) **Gene regulatory peptides**
Gen-regulierende Peptide
Peptides régulant des gènes

(30) Priority: 04.10.2001 EP 01203748; 21.12.2001 US 28075
(43) Date of publication of application: 18.03.2009
(62) Divisional of application: 02763111.8
(73) Proprietor: Biotempt B.V., 7958 NZ Koekange (NL)
(72) Inventor: Khan, Nisar Ahmed, 3039 JL Rotterdam (NL); Benner, Robbert, 2992 SH, Barendrecht (NL)
(74) Representative: Hatzmann, Martin

(56) References cited:
- WO-A-00/32217
- WO-A-01/11048
- WO-A-99/45941
- US-A- 6 159 940
- YAMAMOTO YUMI ET AL: "Role of the NF-kappaB pathway in the pathogenesis of human disease states." CURRENT MOLECULAR MEDICINE (HILVERSUM), vol. 1, no. 3, July 2001 (2001-07), pages 287-296, XP001094465 July, 2001 ISSN: 1566-5240
- WULCZYN F GREGORY ET AL: "The NF-kappa-B/Rel and I-kappa-B gene families: Mediators of immune response and inflammation." JOURNAL OF MOLECULAR MEDICINE (BERLIN), vol. 74, no. 12, 1996, pages 749-769, XP002207478 ISSN: 0946-2716

## Description

In the United States this application is a continuation-in-part of US application 10/028,075 filed December 21, 2001.

Gene control is generally thought to occur at four levels: 1) transcription (either initiation or termination), 2) processing of primary transcripts, 3) stabilization or destabilization of mRNAs, and 4) mRNA translation. The primary function of gene control in cells is to adjust the enzymatic machinery of the cell to its nutritional, chemical and physical environment.

It is generally thought that gene expression is regulated at both the levels of transcription and translation. Modulation or regulation of gene expression requires factors called transcriptional factors. The term "gene control or regulation" also refers to the formation and use of mRNA. Although control can be exerted at a number of different molecular steps, differential transcription probably most frequently underlies the differential rate of protein synthesis in prokaryotes as well as eukaryotes. It is generally thought that activator proteins (also called transcription factors or transcriptional activators) bind to DNA and recruit the transcriptional machinery in a cell to a promotor, thereby stimulating gene expression. Further, differential processing of RNA transcripts in the cell nucleus, differential stabilization of mRNA in the cytoplasm, and differential translation of mRNA into protein are also important in eukaryotic gene control. These steps define the regulatory decisions in a transcriptional circuit and misregulation at any stage can result in a variety of diseases.
Where in unicellular organisms, be it of prokaryotic or eukaryotic origin, a cell's response to its environment is influenced by many stimuli from the outside world, reflecting the often widely variable environment of the single cell, most cells in multicellular organisms experience a fairly constant environment. Perhaps for this reason, genes that are devoted to responses to environmental changes constitute a much smaller fraction of the total number of genes in multicellular organisms than in single-cell organisms.

As said above, cells react to environmental changes, which they perceive through extracellular signals. These signals can be either physical (e.g., light, temperature, pressure and electricity) or chemical (e.g. food, hormones and neurotransmitters). Cells can both sense and produce signals. This makes it possible for them to communicate with each other. In order to achieve this, there are complex signal-sensing and -producing mechanisms in uni- and multi-cellular organisms.

Two groups of chemical signals can be distinguished: membrane-permeable and membrane-impermeable signals. The membrane-permeable signal molecules comprise the large family of steroid hormones, such as estrogens, progesterone and androgens. Steroids pass the plasma membrane and bind to specific receptors, which are localized in the cytoplasm or nucleus of the cell. After binding of the hormone, the receptor undergoes a conformational change. The receptor is then able to bind to DNA itself or to proteins which can in turn interact with DNA. In general, steroid hormones can directly regulate gene expression by means of this process. The membrane-impermeable signal molecules include acetylcholine, growth factors, extracellular matrix components, (peptide)-hormones, neuropeptides, thrombin, lysophosphatidic acid, the yeast mating factors and, for the social amoeba *Dictyostellium discoideum,* folic acid and cyclic AMP. They may be membrane-permeable in themselve but act only outside the cell, i.e. they are recognized by receptors, which are localized on the plasma membrane of the cell. The receptors are specific for one particular signal molecule or a family of closely related signal molecules. Upon binding of their ligands, these receptors transduce the signals by several mechanisms.

The most characteristic and exacting requirement of gene control on multicellular organisms is the execution of precise developmental decisions so that the right gene is activated in the right cell at the right time. These developmental decisions include not only those related to the development of an organism oer se, as for example can be seen during embryogenesis and organogenesis or in response to disease, but also relate to the differentiation or proliferation or apoptosis of those cells that merely carry out their genetic program essentially without leaving progeny behind.

Such cells, such as skin cells, precursors of red blood cells, lens cells of the eye, and antibody-producing cells, are also often regulated by patterns of gene control that serve the need of the whole organism and not the survival of an individual cell.

It is generally reasoned that there are at least three components of gene control: molecular signals, levels and mechanisms. Firstly, it is reasoned that specific signalling molecules exist to which a specific gene can respond. Secondly, control is exerted on one or more levels (i.e., the step or steps) in the chain of events leading from the transcription of DNA to the use of mRNA in protein synthesis. Thirdly, at each of those levels, specific molecular mechanisms are employed to finally exert the control over the gene to be expressed.

Many genes are regulated not by a signalling molecule that enters the cells but by molecules that bind to specific receptors on the surface of cells. Interaction between cell-surface receptors and their ligands can be followed by a cascade of intracellular events including variations in the intracellular levels of so-called second messengers (diacylglycerol, Ca²⁺, cyclic nucleotides). The second messengers in turn lead to changes in protein phosphorylation through the action of cyclic AMP, cyclic GMP, calcium-activated protein kinases, or protein kinase C, which is activated by diaglycerol.

Many of the responses to binding of ligands to cell-surface receptors are cytoplasmatic and do not involve immediate gene activation in the nucleus. Some receptor-ligand interactions, however, are known to cause prompt nuclear transcriptional activation of a specific and limited set of genes. For example, one proto-oncogene, *c-fos,* is known to be activated in some cell types by elevation of almost every one of the known second messengers and also by at least two growth factors, platelet-derived growth factor and epidermal growth factor. However, progress has been slow in determining exactly how such activation is achieved. In a few cases, the transcriptional proteins that respond to cell-surface signals have been characterized.

One of the clearest examples of activation of a pre-existing inactive transcription factor following a cell-surface interaction is the nuclear factor (NF)-kappaB, which was originally detected because it stimulates the transcription of genes encoding immunoglobulins of the kappa class in B-lymphocytes. The binding site for NK-kappaB in the kappa gene is well defined (see for example P.A. Baeuerle and D. Baltimore, 1988, Science 242:540), providing an assay for the presence of the active factor. This factor exists in the cytoplasm of lymphocytes complexed with an inhibitor. Treatment of the isolated complex in vitro with mild denaturing conditions dissociates the complex, thus freeing NK-kappaB to bind to its DNA site. Release of active NF-kappaB in cells is now known to occur after a variety of stimuli including treating cells with bacterial lipopolysaccharide (LPS) and extracellular polypeptides as well as chemical molecules (e.g. phobol esters) that stimulate intracellular phosphokinases. Thus a phosphorylation event triggered by many possible stimuli may account for NF-kappaB conversion to the active state. The active factor is then translocated to the cell nucleus to stimulate transcription only of genes with a binding site for active NF-kappaB.

The inflammatory response involves the sequential release of mediators and the recruitment of circulating leukocytes, which become activated at the inflammatory site and release further mediators (Nat. Med. 7:1294;2001). This response is self-limiting and resolves through the release of endogenous anti-inflammatory mediators and the clearance of inflammatory cells. The persistent accumulation and activation of leukocytes is a hallmark of chronic inflammation. Current approaches to the treatment of inflammation rely on the inhibition of pro-inflammatory mediator production and of mechanisms that initiate the inflammatory response. However, the mechanisms by which the inflammatory response resolves might provide new targets in the treatment of chronic inflammation. Studies in different experimental models of resolving inflammation have identified several putative mechanisms and mediators of inflammatory resolution. We have shown that cyclopentenone prostaglandins (cyPGs) may be endogenous anti-inflammatory mediators and promote the resolution of inflammation *in vivo.* Others have shown a temporal shift to the production of anti-inflammatory lipoxins during the resolution of inflammation. In recent years, apoptosis has been identified as an important mechanism for the resolution of inflammation *in vivo.* It has been postulated that defects in leukocyte apoptosis are important in the pathogenesis of inflammatory disease. In addition, the selective induction of apoptosis in leukocytes may offer a new therapeutic approach to inflammatory disease.

Considering that NF-kappaB is thought by many to be a primary effector of disease (A.S. Baldwin, J. Clin. Invest., 2001, 107:3-6), numerous efforts are underway to develop safe inhibitors of NF-kappaB to be used in treatment of both chronic and acute disease situations. Specific inhibitors of NF-kappaB should reduce side effects associated with drugs such as NSAIDS and glucocorticoids and would offer significant potential for the treatment of a variety of human and animal diseases. Specific diseases or syndromes where patients would benefit from NF-kappaB inhibition vary widely and range from rheumatoid arthritis, atherosclerosis, multiple sclerosis, chronic inflammatory demyelinating polyradiculoneuritis, asthma, inflammatory bowel disease, to Helicobacter pylori-associated gastritis and other inflammatory responses, and a variety of drugs that have effects on NF-kappaB activity, such as corticosteroids, sulfasalazine, 5-aminosalicylic acid, aspirin, tepoxalin, leflunomide, curcumin, antioxidants and proteasome inhibitors. These drugs are considered to be non-specific and often only applicable in high concentrations that may end up toxic for the individual treated.

Inactive cytoplasmatic forms of transcription factors can thus be activated by removal of an inhibitor, as is the case with NF-kappaB, or, alternatively, by association of two (or more) proteins, neither of which is active by itself as in the case of interferon-alpha-stimulated factor (D.E. Levy et al., 1989, Genes and Development 3:1362). After interferon-alpha attaches to its cell-surface receptor, one of the proteins is changed within a minute or less, and the two can combine. The active (combined) factor is then translocated to the cell nucleus to stimulate transcription only of genes with a binding site for the protein. Considering that interferon-alpha is a mediator of responses of the body directed at pathogens and self-antigens, modulating regulation of genes that are under influence of the interferon-alpha-stimulated factor would contribute to the treatment of a variety of human and animal diseases.

Other typical examples of signalling molecules that affect gene expression via cell-surface receptor interaction are polypeptide hormones such as insulin, glucagon, various growth factors such as EGF, VEGF, and so on.

The steroid hormones and their receptors represent one of the best understood cases that affect transcription. Because steroid hormones are soluble in lipid membranes, they can diffuse into cells. They affect transcription by binding to specific intracellular receptors that are site-specific DNA-binding molecules. Other examples of signalling molecules that enter the cell and act intra-cellularly are thyroid hormone (T₃), vitamin D and retinoic acid, and other small lipid-soluble signalling molecules that enter cells and modulate gene expression. The characteristic DNA-binding sites for the receptors for these signalling molecules arc also known as response elements.

Another example of a small molecule that is involved in regulation of gene expression is ethylene, a gas that for example induces the expression of genes involved in fruit ripening. Also, small plant hormones, known as auxines and cytokinins regulate plant growth and differentiation directly by regulating gene expression.

Given the critical role of regulatory factors in gene regulation, the development of artificial or synthetic counterparts that could be used in methods to rectify errors in gene expression has been a long-standing goal at the interface of chemistry and biology.

The inventors have now unearthed an insight in the biology and physiology of the nature of regulatory factors in gene regulation in cellular organisms that allows for an unexpected fast progress in the identification and development of an artificial or synthetic compound acting as a gene regulator, and its use as new chemical entity for the production of a pharmaceutical composition or its use in the treatment of disease. The insight is herein provided that many of small peptides that are derivable by proteolytic breakdown of endogenous proteins of an organism, or that are derivable by proteolytic breakdown of proteins of a pathogen, i.e. during the presence of said pathogen in a host organism, can exert an often very specific gene regulatory activity on cells of said organism.

That small peptides, and even breakdown products, can have biological activity, is already known. Proteolytic breakdown products of endogenous or pathogen derived proteins are for example routinely generated by the proteasome system and presented in the context of class I or II major histocompabilility complex (MHC). Also, it has been recognized that classically known neuropeptides (also known as peptide neurotransmitters) or small peptide hormones, such as antidiuretic hormone, oxytocin, thyrotropin-releasing hormone, gonadotropin-releasing hormone, somatostatinsgastrin, cholecystokinin, substance-P, enkephalins, neurotensin, angiotensins, and derivatives or equivalents thereof have distinct biological activity which is in general mediated by cell-surface receptor interaction. Furthermore, it is now known that certain small and arginine- or lysine- or proline-rich peptides, i.e. having more than 50% of arginine, or 50% of lysine or 50% of proline, or having more than 50% arginine and lysine, or more than 50% arginine and proline, or more than 50% lysine and proline, or more than 50% arginine and lysine and proline residues, have distinct membrane-permeation properties that may result in biological activity.

However, the present invention relates to small peptides other than classically known neuropeptides or peptide hormones, and other than the above identified arginine- or lysine- or proline-rich peptides. Provided is an isolated peptide consisting of the amino acid sequence AQG, herein also referred to as NMPF-43. Also provided is an isolated peptide consisting of the amino acid sequence AQG for use as medicament. In a further aspect, the invention provides a pharmaceutical preparation comprising as an active component an isolated peptide consisting of the amino acid sequence AQG. Also provided is peptide AQG for the treatment of sepsis or for inhibiting angiogenesis.

Synthetic peptides can be obtained using various procedures known in the art. These include solid phase peptide synthesis (SPPS) and solution phase organic synthesis (SPOS) technologies. SPPS is a quick and easy approach to synthesize peptides and small proteins. The C-terminal amino acid is typically attached to a cross-linked polystyrene resin via an acid labile bond with a linker molecule. This resin is insoluble in the solvents used for synthesis, making it relatively simple and fast to wash away excess reagents and byproducts.

Generally, an amino acid consists of a central carbon atom (called the a-carbon) that is surrounded by four other groups: a hydrogen, an amino group, carboxyl group, and a side chain group. The side chain group, designated R, defines the different structures of the amino acids. Certain side chains contain functional groups that can interfere with the formation of the amide bond. Therefore, it is important to mask the functional groups of the amino acid side chain. The N-terminus can be protected with a Fmoc or Boc group, which is stable in acid, but removable by base. Any side chain functional groups are protected with base stable, acid labile groups. To begin each coupling, the masking group on the resin bound amino acid/peptide is removed with 20% piperidine in N,N-dimethyl formamide (DMF). It is then rinsed and a protected amino acid is added which has been activated at its 'alpha' carboxyl group. The activation is achieved by creating the N-hydroxybenzotriazole (HOBt) ester in situ. The activated amino acid and the resin bound amico acid are allowed to react in the presence of base to form a new peptide bond. This process is repeated until the desired peptide is assembled at the resin. Once the peptide is complete, it is ready to be cleaved from the resin. This is accomplished using a mixture of trifluoroacetic acid (TFA) and scavangers. Scavangers serve to neutralize cations which are formed during the removal of the side chain protecting groups. The solution is at least 82% TFA, and the rest a mixture of phenol, thioanisol, water, ethanedithiol (EDT), and triisopropylsilane (TIS). The lead peptide on the resin is allowed to react with the cleavage mixture for several hours, which then affords the peptide in solution. It can then be precipitated and washed in tert-butyl methyl ether, and analyzed or purified as desired.

The thus developed chemical entity can be administered and introduced in-vivo systemically, topically, or locally. The peptide, or is modification or derivative, can be administered as the entity as such or as a pharmaceutically acceptable acid- or base-addition salt, formed by reaction with an inorganic acid (such as hydrochloric acid, hydrobromic acid, perchloric acid, nitric acid, thiocyanic acid, sulfuric acid, and phosphoric acid); or with an organic acid (such as formic acid, acetic acid, propionic acid, glycolic acid, lactic acid, pyruvic acid, oxalic acid, malonic acid, succinic acid, maleic acid, and fumaric acid); or by reaction with an inorganic base (such as sodium hydroxide, ammonium hydroxide, potassium hydroxide); or with an organic base (such as mono-, di-, trialkyl and aryl amines and substituted ethanolamines). A selected peptide and any of the derived entities may also be conjugated to sugars, lipids, other polypeptides, nucleic acids and PNA; and function in-situ as a conjugate or be released locally after reaching a targeted tissue or organ.

A range of suitable pharmaceutical carriers and vehicles are known conventionally to those skilled in the art. Thus, for parenteral administration, the compound will typically be dissolved or suspended in sterile water or saline.

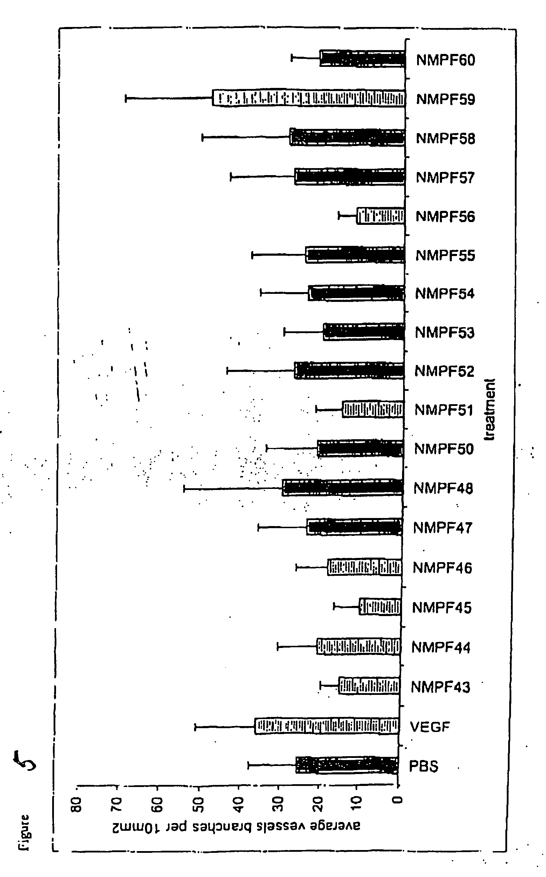

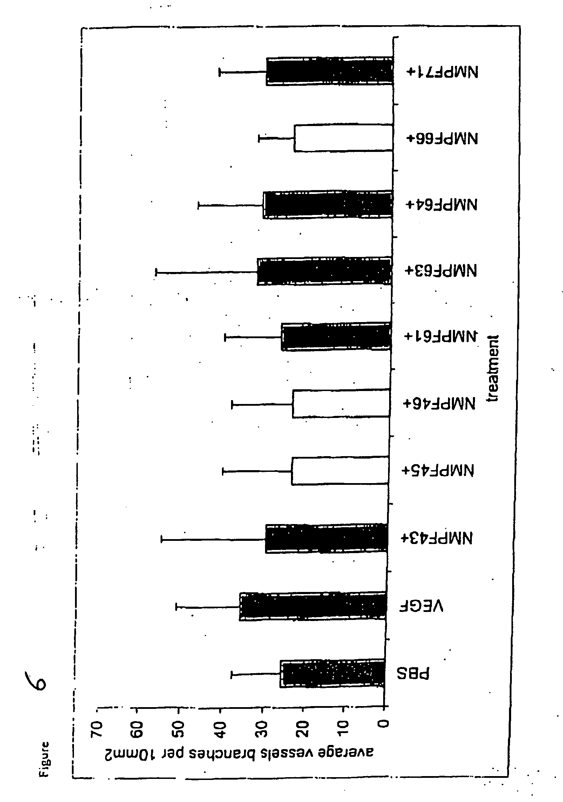

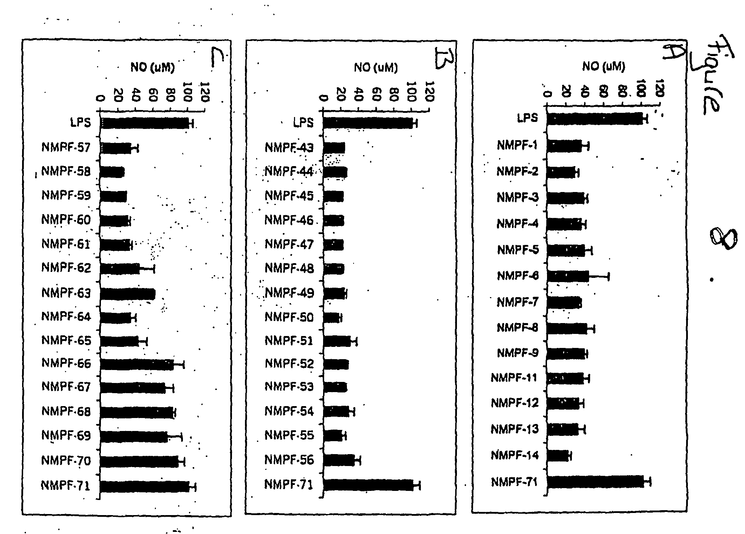

In yet another embodiment, a method is disclosed for modulating angiogenesis, as is illustrated in Fig 5-6 showing the effect of peptide AQG on vessel branching. Also, the invention relates to modulating the process of vasculogenesis as is clearly evidenced by the ability of a peptide to control vessel thickness (Fig 8). It was reported that hCG is a potent angiogeneic factor for uterine endothelial cells in *vitro* and an important role of hCG in endometrial angogenesis was suggested. Importantly, we have now identified hCG-derived peptides that can regulate angiogenesis (see for example table 5) as well as vasculogenisis. Provided is use of AQG for the production of a pharmaceutical composition for the treatment of a large variety of diseases, wherein said peptide is capable of reducing the production of NO and / or TNF alpha in a cell. Diseases include, but are not limited to, septic shock, anthrax, bone disease, arthritis, and ischemic events such as cerebrovascular disease and ischemic heart failure. Also disclosed is a method for treating these diseases comprising administering to a subject in need of such a treatment a molecule comprising an oligopeptide, a peptide or a functional analogue thereof, wherein said molecule is capable of reducing the production NO and / or TNF alpha by a cell. Furthermore, a molecule comprising an oligopeptide, a peptide or a functional analogue thereof, comprises a molecule that is capable of modulating the production of one or more cytokines by a cell. For example, a method for treating these diseases comprises administering to a subject in need of such a treatment a NFkB-regulating peptide.

The invention for example also relates to the treatment of anthrax. Anthrax, the disease caused by the spore-forming *Bacillus anthracis (B. anthracis)*, continues to be a worldwide problem among domesticated and wild herbivores in Asia and Africa and poses a worldwide threat when being used as biological weapons for biological warfare or bioterrorism. Human infections occur after contact with infected animals or contaminated animal products. Outbreaks or epidemics are a constant threat for endemic regions because spores can persist in the soil for long periods of time. Importation controls on certain animal products are necessary to prevent the establishment of anthrax where the disease is not endemic. Human anthrax is usually classified by the portal of entry into the host. Cutaneous anthrax, which accounts for the vast majority of human anthrax cases, is a localized infection with generally mild systemic symptoms and characterized by a painless papule that is surrounded by edema which can be quite extensive. The papule ulcerates by day 5 or 6 and develops into the characteristic black eschar of cutaneous anthrax. Inhalation anthrax, which occurs after inhaling airborne spores, gastrointestinal anthrax, resulting from ingestion of contaminated food, and, in some instances, untreated cutaneous anthrax are characterized by dissemination of the bacteria from the initial site of infection with development of a massive septicemia and toxemia. In inhalation anthrax, phagocytic cells transport the spores from the lung alveoli to the regional lymph nodes, where the spores germinate and bacteria multiply. The bacilli then spread into the bloodstream, where they are temporarily removed by the reticuloendothelial system. Prior to death, which occurs 2 to 5 days after infection, there is a sudden onset of acute symptoms characterized by hypotension, edema, and fatal shock due to an extensive septicemia and toxemia. Therapeutic intervention in general must be initiated early, as septicemic infections are nearly always fatal.

The invention also relates to a method for the treatment of an inflammatory condition comprising administering to a subject in need of such treatment a molecule comprising an oligopeptide peptide or functional analogue or derivative thereof, the molecule capable of reducing production of NO by a cell, in particular wherein the molecule additionally is capable of modulating translocation and/or activity of a gene transcription factor present in a cell, especially wherein the gene transcription factor comprises a NF-kappaB/Rel protein. Advantageously, the invention provides a method.wherein the modulating translocation and/or activity of a gene transcription factor allows modulation of TNF-alpha production by the cell, in particular wherein the TNF-alpha production is reduced. Considering that TNF-alpha production is central to almost all, if not all, inflammatory conditions, reducing TNF-alpha production can greatly alleviate, or mitigate, a great host of inflammatory conditions that are described herein. In particular, the invention discloses a method wherein the inflammatory condition comprises an acute inflammatory condition, and it is especially useful to treat anthrax-related disease, especially when considering that with anthrax, both NO and TNF-alpha reduction will greatly mitigate the course of disease. Table 6 lists oligopeptides according to the invention that have such modulatory effect.

The invention thus discloses a signalling molecule useful in modulating expression of a gene in a cell and/or useful for reducing NO production by a cell. Useful examples of such a signalling molecule can be selected from the group of oligopeptides LQG, AQG, LQGV, AQGV, LQGA, VLPALPQVVC, VLPALP, ALPALP, VAPALP, ALPALPQ, VLPAAPQ, VLPALAQ, LAGV, VLAALP, VLPALA, VLPALPQ, VLAALPQ, VLPALPA, GVLPALP, GVLPALPQ, LQGVLPALPQVVC, VVCNYRDVRFESIRLPGCPRGVNPVV SYAVALSCQCAL, RPRCRPINATLAVEK, EGCPVCITVNTTICAGYCPT, SKAPPPSLPSPSRLPGPS, SIRLPGCPRGVNPVVS, LPGCPRGVNPVVS, LPGC, MTRV, MTR, VVC, QVVC and functional analogues or derivatives thereof.

Furthermore, the invention provides use of oligopeptide AQG for the production of a pharmaceutical composition for the treatment of an inflammatory condition or a post-meno-pausel condition, or a bone disease such as osteoporosis, or for the induction of weight loss. The term "pharmaceutical composition" as used herein is intended to cover both the active signalling molecule alone or a composition containing the signalling molecule together with a pharmaceutically acceptable carrier, diluent or excipient. Acceptable diluents of an oligopeptide as described herein in the detailed description are for example physiological salt solutions or phosphate buffered salt solutions. In one embodiment of the present invention, AQG is administered in an effective concentration to an animal or human systemically, e.g. by intravenous, intra-muscular or intraperitoneal administration. Another way of administration comprises perfusion of organs or tissue, be it *in vivo* or *ex vivo*, with a perfusion fluid comprising a signal molecule according to the invention. Topical administration, e.g. in ointments or sprays, may also apply, e.g. in inflammations of the skin or mucosal surfaces of for example mouth, nose and/or genitals. Local administration can occur in joints, bursae, tendon sheaths, in or around the spinal cord at locations where nerve bundles branch off, at the location of hernias, in or around infarcted areas in brain or heart, etc. The administration may be done as a single dose, as a discontinuous sequence of various doses, or continuously for a period of time sufficient to permit substantial modulation of gene expression. In the case of a continuous administration, the duration of the administration may vary depending upon a number of factors which would readily be appreciated by those skilled in the art.

The administration dose of the active molecule may be varied over a fairly broad range. The concentrations of an active molecule which can be administered would be limited by efficacy at the lower end and the solubility of the compound at the upper end. The optimal dose or doses for a particular patient should and can be determined by the physician or medical specialist involved, taking into consideration well-known relevant factors such as the condition, weight and age of the patient, etc.

The active molecule may be administered directly in a suitable vehicle, such as e.g. phosphate-buffered saline (PBS) or solutions in alcohol or DMSO. Pursuant to preferred embodiments of the present invention, however, the active molecule is administered through a single dose delivery using a drug-delivery system, such as a sustained-release delivery system, which enables the maintenance of the required concentrations of the active molecule for a period of time sufficient for adequate modulation of gene expression. A suitable drug-delivery system would be pharmacologically inactive or at least tolerable. It should preferably not be immunogenic nor cause inflammatory reactions, and should permit release of the active molecule so as to maintain effective levels thereof over the desired time period. A large variety of alternatives are known in the art as suitable for purposes of sustained release and are contemplated as within the scope of the present invention. Suitable delivery vehicles include, but are not limited to, the following: microcapsules or microspheres; liposomes and other lipid-based release systems; viscous instillates; absorbable and/or biodegradable mechanical barriers and implants; and polymeric delivery materials, such as polyethylene oxide/polypropylene oxide block copolymers, polyesters, cross-linked polyvinylalcohols, polyanhydrides, polymethacrylate and polymethacrylamide hydrogels, anionic carbohydrate polymers, etc. Useful delivery systems are well known in the art.

A highly suitable formulation to achieve the active molecule release comprises injectable microcapsules or microspheres made from a biodegradable polymer, such as poly(dl-lactide), poly(dl-lactide-co-glycolide), polycaprolactone, polyglycolide, polylactic acid-co-glycolide, poly(hydroxybutyric acid), polyesters or polyacetals. Injectable systems comprising microcapsules or microspheres having a diameter of about 50 to about 500 micrometers offer advantages over other delivery systems. For example, they generally use less active molecules and may be administered by paramedical personnel. Moreover, such systems are inherently flexible in the design of the duration and rate of separate drug release by selection of microcapsule or microsphere size, drug loading and dosage administered. Further, they can be successfully sterilized by gamma irradiation.

The design, preparation and use of microcapsules and microspheres are well within the reach of persons skilled in the art and detailed information concerning these points is available in the literature. Biodegradable polymers (such as lactide, glycolide and caprolactone polymers) may also be used in formulations other than microcapsules and microspheres; e.g. premade films and spray-on films of these polymers containing the active molecule would be suitable for use in accordance with the present invention. Fibers or filaments comprising the active molecule are also contemplated as within the scope of the present invention.

Another highly suitable formulation for a single-dose delivery of the active molecule in accordance with the present invention involves liposomes. The encapsulation of an active molecule in liposomes or multilamellar vesicles is a well-known technique for targeted drug delivery and prolonged drug residence. The preparation and use of drug-loaded liposomes is well within the reach of persons skilled in the art and well documented in the literature.

Yet another suitable approach for single-dose delivery of an active molecule in accordance with the present invention involves the use of viscous installates. In this technique, high molecular weight carriers are used in admixture with the active molecule, giving rise to a structure which produces a solution with high viscosity. Suitable high molecular weight carriers include, but are not limited to, the following: dextrans and cyclodextrans; hydrogels; (cross-linked) viscous materials, including (cross-linked) viscoelastics; carboxymethylcellulose; hyaluronic acid; and chondroitin sulfate. The preparation and use of drug-loaded viscous instillates is well known to persons skilled in the art.

Pursuant to yet another approach, the active molecule may be administered in combination with absorbable mechanical barriers such as oxidized regenerated cellulose. The active molecule may be covalently or non-covalently (e.g., ionically) bound to such a barrier, or it may simply be dispersed therein.

A pharmaceutical composition as provided herein is particularly useful for the modulation of gene expression by inhibiting NF-kappaB/Rel protein activation.

NF-kappaB/Rel proteins are a group of structurally related and evolutionarily conserved proteins (Rel). Well known are c-Rel, RelA (p65), RelB, NF-kappaB1 (p50 and its precursor p105), and NF-kappaB2 (p52 and its precursor p100). Most NF-kappaB dimers are activators of transcription; p50/p50 and p52/p52 homodimers repress the transcription of their target genes. All NF-kappaB/Rel proteins share a highly conserved NH2-terminal Rel homology domain (RHD). RHD is responsible for DNA binding, dimerization, and association with inhibitory proteins known as IkappaBs. In resting cells, NF-kappaB/Rel dimers are bound to IkappaBs and retained in an inactive form in the cytoplasm. IkappaBs are members of a multigene family (IkappaBalpha, IkappaBbeta, IkappaBgamma, IkappaBepsilon, Bcl-3, and the precursor Rel-proteins, p100 and p105. Presence of multiple copies of ankyrin repeats interact with NF-kappaB via the RHD (protein-protein interaction. Upon appropriate stimulation, IkappaB is phosphorylated by IkappaB Kinase (IKKs), polyubiquitinated by ubiquitin ligase complex, and degraded by the 26S proteosome. NF-kappaB is released and translocates into nucleus to initiate gene expression.

NF-kappaB regulation of gene expression includes innate immune responses: such as regulated by cytokines IL-1, IL-2, IL-6, IL-12, TNF-alpha, LT-alpha, LT-beta, GM-CSF; expression of adhesion molecules (ICAM, VCAM, endothelial leukocyte adhesion molecule [ELAM]), acute phase proteins (SAA), iInducible enzymes (iNOS and COX-2) and antimicrobial peptides (beta-defensins). For adaptive immunity, MHC proteins IL-2, IL-12 and IFN-alpha are regulated by NF-kappaB. Regulation of overall immune response includes the regulation of genes critical for regulation of apoptosis (c-IAP-1 and c-IAP-2, Fas Ligand, c-myc, p53 and cyclin D1.

Several oligopeptides capable of modulating gene expression disclosed herein have earlier been tested, both ex vivo and in vivo, and in small animals, but a relationship with modulation of gene expression was not brought forward. A beneficial effect of these oligopeptides on LPS-induced sepsis in mice, namely the inhibition of the effect of the sepsis, was observed. Immunomodulatory effects with these oligopeptides have been observed in *vitro* and in *ex vivo* such as in T-cell assays showing the inhibition of pathological Th1 immune responses, suppression of inflammatory cytokines (MIF), increase in production of anti-inflammatory cytokines (IL-10, TGF-beta) and immunomodulatory effects on antigen-presenting cells (APC) like dendritic cells, monocytes and macrophages.

Now that the insight has been provided that distinct synthetic oligopeptides or functional analogues or derivatives thereof, for example those that resemble breakdown products which can be derived by proteolysis from endogenous proteins such as hCG, can be used to modulate gene expression, for example by NF-kappaB inhibition, such oligopeptides find much wider application. Release of active NF-kappaB in cells is now known to occur after a variety of stimuli including treating cells with bacterial lipopolysaccharide (LPS) and the interaction with a Toll-like receptor (see for example Guha and Mackman, Cell. Sign. 2001, 13:85-94). In particular, LPS stimulation of dendritic cells, monocytes and macrophages induces many genes that are under the influence of activation by transcription factors such as NF-kappaB, p50, EGR-1, IRF-1 and others that can be modulated by a signalling molecule according to the invention. Considering that LPS induction of EGR-1 is required for maximal induction of TNF-alpha, it is foreseen that inhibition of EGR-1 considerably reduces the effects of sepsis seen after LPS activation. Now knowing the gene modulatory effect of the signalling molecules such as oligopeptides as provided herein allows for rational design of signal molecule mixtures that better alleviate the symptoms seen with sepsis. Accordingly, the invention provides a pharmaceutical composition for the treatment of sepsis in a primate and a method for the treatment of sepsis in a primate comprising subjecting the primate to a signalling molecule according to the invention. Administration of such a signalling molecule preferably occurs systematically, e.g. by intravenous or intraperitoneal administration. In a further embodiment, such treatment also comprises the use of for example an antibiotic, however, only when such use is not contraindicated because of the risk of generating further toxin loads because of lysis of the bacteria subject to the action of those antibiotics in an individual thus treated.

### BRIEF DESCRIPTION OF THE FIGURES

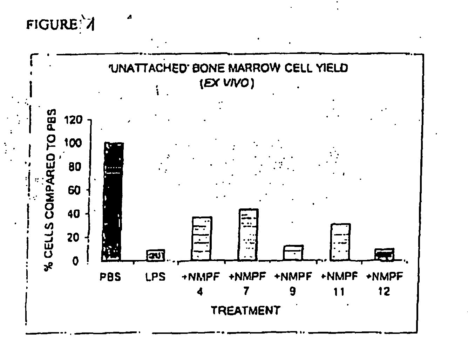

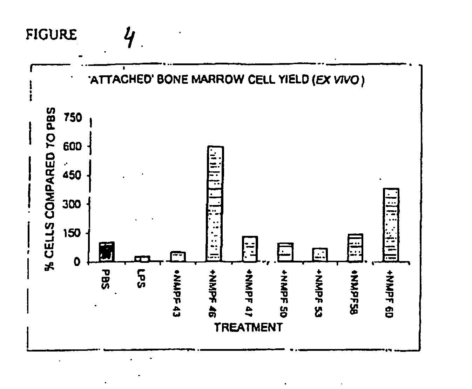

Figure 1-4 Bone marrow (BM) cell yield of treated BALB/c mice (n=6). BM cells were isolated from treated mice and cultured ex vivo in the presence of rmGM-CSF for nine days. These figures show cell yield after nine days of culture of BM cells in suspension (unattached) and attached to Petri dish (attached). BM cells were isolated from mice treated with PBS, LPS or LPS in combination with different NMPF peptides. In these figures cell yield is expressed in relative percentage of cells compared to PBS. Each condition consists of 6 Petri dishes and results shown here are representative of 6 dishes. Differences of ≥ 20% were considered significant and line bars represent significant data as compared to LPS control group. A representative experiment is shown. Findings involving all experimental conditions were entirely reproduced in 3 additional experiments.

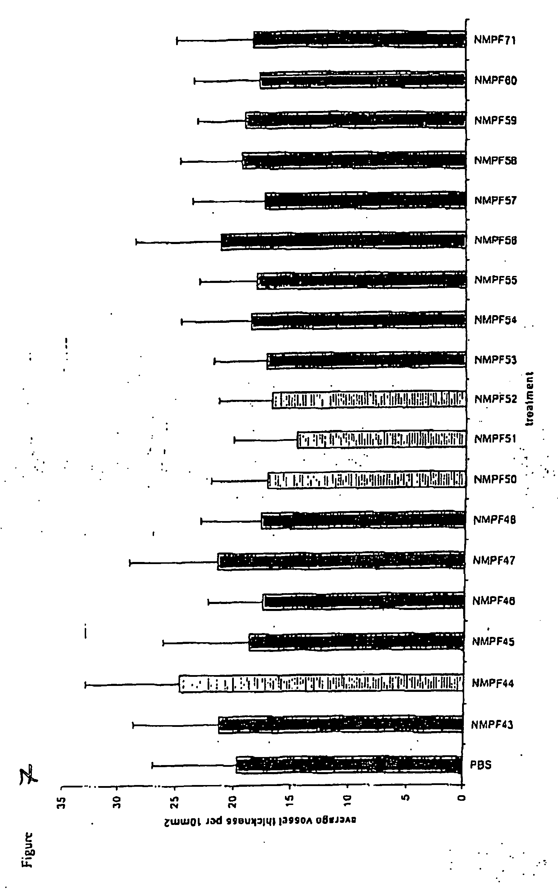

Figures 5-7 In vivo treatment of fertilized chicken eggs with NMPF and the effect of NMPF on angiogenesis. Fertile chicken eggs (day 0) were treated with either PBS, NMPF, VEGF or VEGF in combination with NMPF. Ten eggs were injected for every condition. On day 8 of incubation, the embryos were removed from the eggs and were placed in a 100-mm Petri dish. The embryo and the blood vessels were photographed in vivo with the use of a microscope. Of each egg one overview picture was taken and 4 detail pictures of the blood vessels were taken. Quantification of angiogenesis (vessel branches) was accomplished by counting the number of blood vessel branches. Quantification of this vasculogenesis was accomplished by measuring the blood vessel thickness. The number of blood vessel branches and vessel thichness were measured in the pictures and were correlated to a raster (in the pictures) of 10mm² for comparison. The mean number of branches and the mean blood vessel thickness of each condition (N=10) were calculated and compared to either the PBS or VEGF controls using a Student's T-test. Line bars represent significant (p<0.05) data as compared to PBS control group and dotted bars represent significant (p<0.05) data as compared to VEGF group. Figures 5-6 show the effect of NMPF on vessel branches. Figure 7 shows the effect of NMPF on vessel thickness.
Figure 8 These figures (parts A-C) show the NO production of LPS (10 pg/ml) stimulated RAW 264.7 macrophages co-stimulated with different NMPF peptides (1 pg/ml).

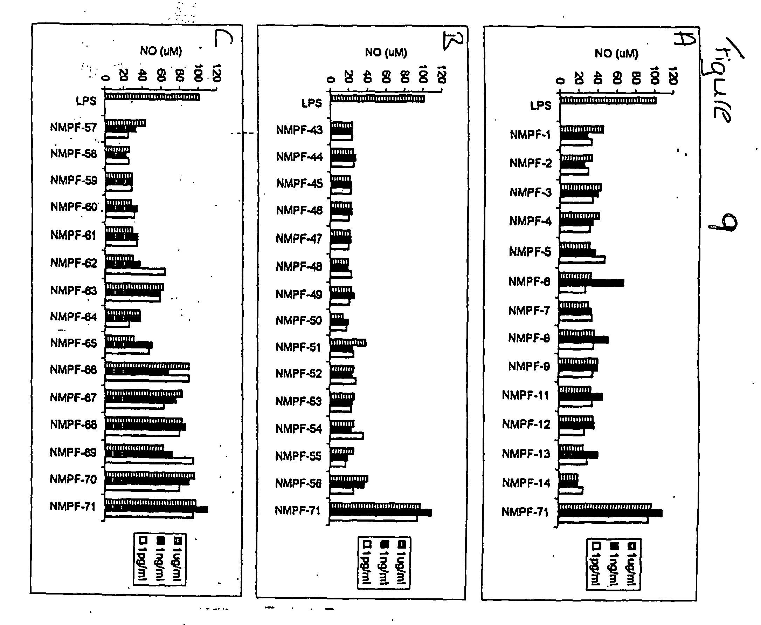

Figure 9 These figures (parts A-C) show the NO production of LPS (10 µg/ml) stimulated RAW 264.7 macrophages co-stimulated with different NMPF peptides with three different concentrations.

The invention is further explained with the aid of the following illustrative examples.

### EXAMPLES

### MATERIAL AND METHODS

### Peptide synthesis

The peptides as mentioned in this document such as LQG, AQG, LQGV, AQGV, LQGA, VLPALP, ALPALP, VAPALP, ALPALPQ, VLPAAPQ, VLPALAQ, LAGV, VLAALP, VLPALA, VLPALPQ, VLAALPQ, VLPALPA, GVLPALP, VVCNYRDVRFESIRLPGCPRGVNPVVSYAVALSCQCAL, RPRCRPINATLAVEKEGCPVCITVNTTICAGYCPT, SKAPPPSLPSPSRLPGPS, LQGVLPALPQVVC, SIRLPGCPRGVNPVVS, LPGCPRGVNPVVS, LPGC, MTRV, MTR, and VVC were prepared by solid-phase synthesis (Merrifield, 1963) using the fluorenylmethoxycarbonyl (Fmoc)/tert-butyl-based methodology (Atherton, 1985) with 2-chlorotrityl chloride resin (Barlos, 1991) as the solid support. The side-chain of glutamine was protected with a trityl function. The peptides were synthesized manually. Each coupling consisted of the following steps: (i) removal of the alpha-amino Fmoc-protection by piperidine in dimethylformamide (DMF), (ii) coupling of the Fmoc amino acid (3 eq) with diisopropylcarbodiimide (DIC)/1-hydroxybenzotriazole (HOBt) in DMF/N-methylformamide (NMP) and (iii) capping of the remaining amino functions with acetic anhydride/diisopropylethylamine (DIEA) in DMF/NMP. Upon completion of the synthesis, the peptide resin was treated with a mixture of trifluoroacetic acid (TFA)/H₂O/triisopropylsilane (TIS) 95:2.5:2.5. After 30 minutes TIS was added until decolorization. The solution was evaporated in vacuo and the peptide precipitated with diethylether. The crude peptides were dissolved in water (50-100 mg/ml) and purified by reverse-phase high-performance liquid chromatography (RP-HPLC). HPLC conditions were: column: Vydac TP21810C18 (10 x 250 mm); elution system: gradient system of 0.1% TFA in water v/v (A) and 0.1% TFA in acetonitrile (ACN) v/v (B); flow rate 6 ml/min; absorbance was detected from 190-370 nm. There were different gradient systems used. For example for peptides LQG and LQGV: 10 minutes 100% A followed by linear gradient 0-10% B in 50 minutes. For example for peptides VLPALP and VLPALPQ: 5 minutes 5% B followed by linear gradient 1% B/minute. The collected fractions were concentrated to about 5 ml by rotation film evaporation under reduced pressure at 40°C. The remaining TFA was exchanged against acetate by eluting two times over a column with anion exchange resin (Merck II) in acetate form. The elute was concentrated and lyophilised in 28 hours. Peptides later were prepared for use by dissolving them in PBS.

### Endotoxin shock model (Sepsis)

Sepsis. For the endotoxin model, BALB/c mice were injected i.p. with 8-9 mg/kg LPS (E. coli 026:B6; Difco Lab., Detroit, MI, USA). Control groups (PBS) were treated with PBS i.p. only. To test the effect of NMPF from different sources (synthetic, commercial hCG preparation [c-hCG]), we treated BALB/c with a dose of 300-700 IU of different hCG preparations (PG23;Pregnyl batch no. 235863, PG25; Pregnyl batch no. 255957) and with synthetic peptides (5 mg/kg) after two hours of LPS injection. In other experiments BALB/c mice were injected i.p. either with 10 mg/kg or with 11 mg/kg LPS (E. coli 026:B6; Difco Lab., Detroit, MI, USA). Subsequently mice were treated after 2 hours and 24 hours of LPS treatment with NMPF peptides.

*Semi-quantitative sickness measurements*. Mice were scored for sickness severity using the following measurement scheme:
1 Percolated fur, but no detectable behaviour differences compared to normal mice.
2 Percolated fur, huddle reflex, responds to stimuli (such as tap on cage), just as active during handling as healthy mouse.
3 Slower response to tap on cage, passive or docile when handled, but still curious when alone in a new setting.
4 Lack of curiosity, little or no response to stimuli, quite immobile.
5 Laboured breathing, inability or slow to self-right after being rolled onto back (moribund)
6 Sacrificed

### RESULTS

### Endotoxin shock model (Sepsis)

*Sepsis experiments.* To determine the effect of synthetic peptides (NMPF) in high-dose LPS shock model, BALB/c mice were injected intraperitoneally with different doses of LPS and survival was assessed daily for 5 days. In this experiment (for the LPS endotoxin model) BALB/c mice were injected i.p. with 8-9 mg/kg LPS (E. coli 026:B6; Difco Lab., Detroit, MI, USA). Control groups (PBS) were treated with PBS i.p. only. We treated BALB/c mice with a dose of 300-700 IU of different hCG preparations (PG23;Pregnyl batch no. 235863, PG25; Pregnyl batch no. 255957) or with peptides (5 mg/kg) after two hours of LPS injection.

These experiments showed (table 1.) that NMPF peptides 4, 6, 66 and PG23 inhibited shock completely (all mice had in first 24 hours sickness scores not higher than 2; shortly thereafter they recovered completely and had sickness scores of 0), while peptides 2, 3 and 7 accelerated shock (all mice had in first 24 hours sickness scores of 5 and most of them died, while the control mice treated with LPS+PBS had sickness scores of 3-4 in first 24 hours and they most of them died after 48 hours with sickness scores of 5 (17% survival rate at 72 hours). In addition, peptides 1, 5, 8, 9, 11, 12, 13, 14 and 64 showed in number of different experiments variability in effectiveness as well as in the kind (inhibitory vs accelerating) of activity. This variability is likely attributable to the rate of breakdown of the various peptides, and the different effects the various peptides and their breakdown products have *in vivo*. In addition, these experiments also showed the variability in anti-shock activity in c-hCG preparations that is likely attributable to the variation in the presence of anti-shock and shock accelerating NMPF. Visible signs of sickness were apparent in all of the experimental animals, but the kinetics and obviously the severity of this sickness were significantly different. These data are representative of at least 10 separate experiments.

In Table 2 we see the effect of ALA-replacement (PEPSCAN) in peptide LQG, LQGV, VLPALP, VLPALPQ in septic shock experiments. We conclude, that the change in even one amino acid by a neutral amino acid can lead to different activity. So, genomic differences as well as polymorphism in these peptides can regulate the immune response very precise. Derivatives of these peptides, for example (but not limited to) by addition of classical and non-classical amino acids or derivatives that are differentially modified during or after synthesis, for example benzylation, amidation, glycosylation, proteolytic cleavage, linkage to an antibody molecule or other cellular ligand etc. could also lead to a better effectiveness of the activity.

To determine whether treatment of BALB/c mice with NMPF inhibit septic shock at different stages of disease, synthetic peptides (NMPF) were injected i.p. at 2 and 24 hours after the induction of septic shock with high dose LPS (10 mg/kg).

As shown in Tables 3 and 4, control mice treated PBS after the shock induction, reached a sickness score of 5 at 14 and 24 hours, and remained so after the second injection with PBS. The survival rate in control group mice was 0% at 48 hours. In contrast to control mice, mice treated with NMPF 9, 11, 12, 43, 46, 50 and 60 reached a maximum sickness score of 2-3 at 24 hours after the induction of septic shock and further reached a maximum sickness score of 1-2 at 48 hours after the second injection of NMPF. In addition, mice treated with NMPF 5, 7, 8, 45, 53 and 58 reached a sickness score of 5 and after the second injection with NMPF all mice returned to a sickness score of 1-2 and survival rates in NMPF groups were 100%. Mice treated with NMPF 3 reached sickness scores of 3-4 and the second NMPF injection did save these mice. These experiments show that NMPF peptides have anti-shock activity at different stages of the disease and NMPF have anti-shock activity even at disease stage when otherwise irreversible damage had been done. This indicates that NMPF have effects on different cellular levels and also have repairing and regenerating capacity.

### Dendritic cells experiments

*Mice*. The mouse strain used in this study was BALB/c (Harlan, Bicester, Oxon, GB). All mice used in experiments were females between 8 and 12 weeks of age.

Mice were housed in a specific-pathogen-free facility. The Animal Use Committee at the Erasmus University Rotterdam, The Netherlands approved all studies.

*In vivo treatment.* At least six mice per group were injected intraperitonally (i.p) with LPS (10 mg/kg; Sigma). After 2 and 24 hrs of LPS induction, mice were injected i.p. with either NMPF (5 mg/kg) or Phosphate Buffered Saline (PBS), in a volume of 100 µl. LPS induced shock in this model had more than 90% mortality at 48 hrs.

*Bone marrow cell culture.* From treated mice, bone-marrow cells were isolated and cultured as follows. BALB/c mice were killed by suffocation with CO₂. The femurs and tibiae were removed and freed of muscles and tendons under aseptic conditions. The bones were placed in R10 medium (RPMI 1640, supplemented with 50 U/ml penicillin, 50 µg/ml streptomycin, 0.2 M Na-pyruvate, 2 mM glutamine, 50 µM 2-mercaptoethanol and 10% fetal calf serum (Bio Whittaker, Europe)).

The bones were then cleaned more thoroughly by using an aseptic tissue and were transferred to an ice cold mortier with 2 ml of R10 medium. The bones were crushed with a mortel to get the cells out of the bones. Cells were filtered through a sterile 100 µM filter (Beckton Dickinson Labware) and collected in a 50 ml tube (FALCON). This procedure was repeated until bone parts appeared translucent.

The isolated cells were resuspended in 10 ml of R10 and 30 ml of Geys medium was added. The cell suspension was kept on ice for 30 minutes, to lyse the red blood cells. Thereafter, the cells were washed twice in R10 medium. Upon initiation of the culture, the cell concentration was adjusted to 2 x 10⁵ cells per ml in R10 medium supplemented with 20 ng/ml recombinant mouse Granulocyte Monocyte-Colony Stimulating Factor (rmGM-CSF; BioSource International, Inc., USA) and seeded in 100 mm non-adherent bacteriological Petri dishes (Falcon). For each condition six Petri dishes were used and for further analysis, cells were pooled and analysed as described ahead. The cultures were placed in a 5% CO₂-incubator at 37°C. Every three days after culture initiation, 10 ml fresh R10 medium supplemented with rmGM-CSF at 20 ng/ml was added to each dish.

Nine days after culture initiation, non-adherent cells were collected and counted with a Coulter Counter (Coulter).

Alternatively, BM cells from untreated mice were isolated and cultured as described above and were in *vitro* treated with the following conditions: NMPF 4, NMPF 46, NMPF 7, NMPF 60 (20 µg/ml) were added to the culture either at day 0 or day 6 after culture initiation. Or LPS (1 µg/ml) was added to the culture at day 6 with or without the NMPF.

Immunofluorescence staining. Cells (2 x 10⁵) were washed with FACS-buffer (PBS with 1% BSA and 0.02% sodium azide), and transferred to a round-bottomed 96-well plate (NUNC). The antibodies used for staining were against MHC-II (I-A/I-E) PE and CD11c/CD18 FITC (PharMingen/Becton Dickinson, Franklin Lakes, NJ, US).

Cells were resuspended in 200 µl FACS-buffer containing both of the antibodies at a concentration of 2.5 ng/µl per antibody. Cells were then incubated for 30 min at 4°C. Thereafter, cells were washed 3 times and finally resuspended in 200 µl FACS-buffer for flow-cytometric analysis in a FACSCalibur flow cytometer (Becton Dickinson, Heidelberg, Germany). All FACS-data were analyzed with CellQuest software (Becton Dickinson, Heidelberg, Germany).

*Statistical analysis* All differences greater than 20% are considered to be significant.

### RESULTS

### Dendritic cells experiments

*Cell yields of in vitro bone-marrow cell cultures.* To determine the effect of LPS and NMPF *in vitro* on the cell yield of a nine-day culture of bone-marrow cells, we isolated the BM cells from untreated BALB/c mice and cultured in the presence of rmGM-CSF. In addition to rmGM-CSF, cultures were supplemented with NMPF at either day 0 or day 6 with or without the addition of LPS at day 6.

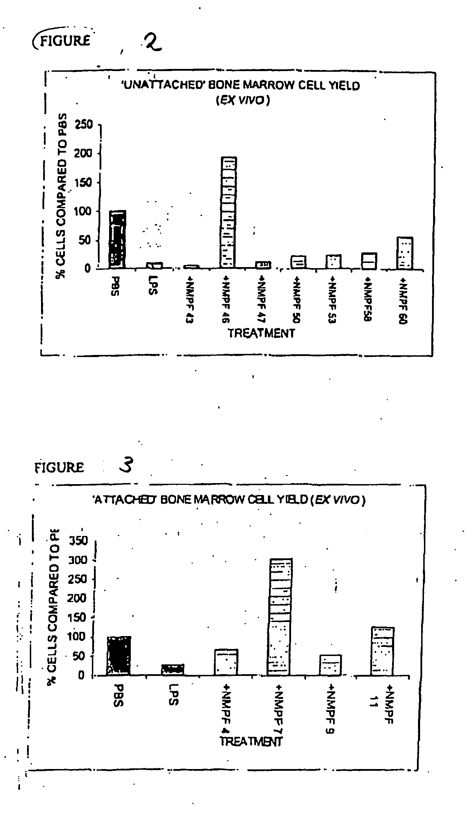

The cell yield of 'attached' cells was significant increased with NMPF 4, 7, 9, 11, 43, 46, 47, 50, 53, 58 60 and even in the group of NMPF 7, 46 and 60 the cell yield was significant increased as compared to the PBS group (figure 3-4). In addition, cell yield of 'un-attached' cells was significant increased with NMPF 4, 7, 9, 11, 46, 50, 53, 58 60 and again in the group of NMPF 46 the cell yield was significant increased as compared to the PBS group (figure 1-2).

### RESULTS

### Chicken eggs experiments

*In vivo treatment of fertilised chicken eggs with NMPF.* Fertile chicken eggs (Drost Loosdrecht BV, the Netherlands) were incubated in a diagonal position in an incubator (Pas Reform BV, the Netherlands) at 37 °C and 32% relative humidity.

Solutions of NMPF peptides (1mg/ml) and VEGF were made in PBS. At least ten eggs were injected for every condition. The treatment was performed as follows: on day 0 of incubation, a hole was drilled into the eggshell to open the air cell. A second hole was drilled 10 mm lower and right from the first hole for injection. The holes in the eggshell were disinfected with jodium. The NMPF peptides (100 ug/egg) and/or VEGF (100 ng/ml) were injected in volume of 100µl. The holes in the eggshell were sealed with tape (Scotch Magic™ Tape, 3M) and the eggs were placed into the incubator.

*Quantification of angiogenesis.* On day 7 of incubation, the eggs were viewed under an UV lamp to check if the embryos were developing in a normal way and the dead embryos were counted. On day 8 of incubation, the embryos were removed from the eggs by opening the shell at the bottom of the eggs. The shell membrane was carefully dissected and removed. The embryos were placed in a 100-mm Petri dish. The embryo and the blood vessels were photographed (Nikon E990, Japan) *in vivo* with the use of a microscope (Zeiss Stemi SV6, Germany). One overview picture was taken and 4 detail pictures of the blood vessels were taken. Only eggs with vital embryos were evaluated.

*Data analysis.* Quantification of angiogenesis was accomplished by counting the number of blood vessels branches. Quantification of vasculogenesis was accomplished by measuring the blood vessel thickness. The number of blood vessel branches and the blood vessel thickness were counted in the pictures (4 pictures/egg) using Corel Draw 7. Thereafter, the number of blood vessel branches and the thickness of the blood vessels were correlated to a raster of microscope (10 mm²) for comparison. The mean number of branches and the mean blood vessel thickness of each condition (n=10) were calculated and compared to the PBS control eggs using a Student's T-test.

### RESULTS

### Chicken eggs experiments

In order to determine the effect of NMPF on angiogenesis and vasculogenesis we treated fertilized chicken eggs with NMPF or NMPF in combination with VEGF as described in materials and methods section. Figures 5-6 show that NMPF 3, 4, 9 and 11 promoted angiogenesis (p<0.05), while NMPF VEGF, 7, 43, 44, 45, 46, 51 and 56 inhibited angiogenesis (p<0.05). NMPF 6, 7, 12, 45, 46 and 66 were able to inhibit angiogenesis induced by VEGF. Moreover, NMPF 6 itself did not show any effect on angiogensis, but it inhibited (p<0.05) NMPF 3 induced angiogenesis.

Figure 7 shows that NMPF 1, 2,3, 4, 6, 7, 8, 12, 50, 51, and 52 had vasculogenesis inhibiting (p<0.05) effect, while only NMPF 44 promoted (p<0.05) vasculogenesis.

### NO experiment

*Cell culture.* The RAW 264.7 murine macrophage cell line, obtained from American Type Culture Collection (Manassas, VA, USA), were cultured at 37°C in 5% CO₂ using DMEM containing 10% fetal calf serum (FCS), 50 U/ml penicillin, 50 µg/ml streptomycin, 0.2 M Na-pyruvate, 2 mM glutamine and 50 µM 2-mercaptoethanol (Bio Whittaker, Europe). The medium was changed every 2 days.

*Nitrite measurements.* Nitrite production was measured in the RAW 264.7 macrophage supernatants. The cells (7.5 x10⁵/ml) were cultured in 48-well plates in 500 microliter of culture medium. The cells were stimulated with LPS (10 microg/ml) and/or NMPF (1 pg/ml, 1 ng/ml, 1 microg/ml) for 24 hours, then the culture media were collected. Nitrite was measured by adding 100 microl of Griess reagent (Sigma) to 100 microl samples of culture medium. The OD₅₄₀ was measured using a microplate reader, and the nitrite concentration was calculated by comparison with the OD₅₄₀ produced using standard solutions of sodium nitrite in the culture medium.

### RESULTS

### NO experiment

NO production is a central mediator of the vascular and inflammatory response. Our results show that macrophages (RAW 264.7) stimulated with LPS produce large amount of NO. However, these cells co-stimulated with most of the NMPF peptides (NMPF peptide 1 to 14, 43 to 66 and 69) even in a very low dose (1 pg/ml) inhibited the production of NO. (Fig. 8)

**TABLE 1. Results of shock experiments in mice**

| TEST SUBSTANCE | | % SURVIVAL IN TIME | | | |
|---|---|---|---|---|---|
| (HRS) | | 0 | 16 | 40 | 72 |
| PBS | | 100 | 100 | 67 | 17 |
| PG23 | | 100 | 100 | 100 | 100 |
| PG25 | | 100 | 83 | 83 | 83 |
| | | | | | |

| PEPTIDE | | | | | |
|---|---|---|---|---|---|
| NMPF | SEQUENCE | | | | |
| 1 | VLPALPQVVC | 100 | 100 | 50 | 17 |
| 2 | LQGVLPALPQ | 100 | 67 | 0 | 0 |
| 3 | LQG | 100 | 83 | 20 | 17 |
| 4 | LQGV | 100 | 100 | 100 | 100 |
| 5 | GVLPALPQ | 100 | 100 | 80 | 17 |
| 6 | VLPALP | 100 | 100 | 100 | 100 |
| 7 | VLPALPQ | 100 | 83 | 0 | 0 |
| 8 | GVLPALP | 100 | 100 | 83 | 67 |
| 9 | VVC | 100 | 100 | 50 | 50 |
| 11 | MTRV | 100 | 100 | 67 | 50 |
| 12 | MTR | 100 | 100 | 67 | 50 |
| 13 | LQGVLPALPQVVC | 100 | 100 | 100 | 100 |
| 14 | (CYCLIC) LQGVLPALPQVVC | 100 | 83 | 83 | 83 |
| 64 | LPGCPRGVNPVVS | 100 | 100 | 100 | 100 |
| 66 | LPGC | 100 | 100 | 100 | 100 |

**TABLE 2. Additional results of shock experiments**

| NMPF SEQUENCE ID: | ANTI-SHOCK EFFECT |
|---|---|
| | |
| LQGV | +++ |
| AQGV | +++ |
| LQGA | +++ |
| VLPALP | +++ |
| ALPALP | ++ |
| VAPALP | ++ |
| ALPALPQ | ++ |
| VLPAAPQ | ++ |
| VLPALAQ | +++ |

| | SHOCK ACCELERATING EFFECT |
|---|---|
| LAGV | +++ |
| LQAV | +++ |
| VLAALP | +++ |
| VLPAAP | +++ |
| VLPALA | +++ |
| VLPALPQ | +++ |
| VLAALPQ | +++ |
| VLPALPA | +++ |

**TABLE 3. Further additional results of shock experiments**

| NMPF PEPTIDES | | % SURVIVAL IN TIME (HRS) | | |
|---|---|---|---|---|
| | | | | |

| Tx | Tx | | | |
|---|---|---|---|---|
| | 0 | 14 | 24 | 48 |
| PBS | 100 | 100 | 100 | 0 |
| NMPF-3 | 100 | 100 | 100 | 0 |
| NMPF-5 | 100 | 100 | 100 | 100 |
| NMPF-7 | 100 | 100 | 100 | 67 |
| NMPF-8 | 100 | 100 | 100 | 100 |
| NMPF-9 | 100 | 100 | 100 | 100 |
| NMPF-11 | 100 | 100 | 100 | 100 |
| NMPF-12 | 100 | 100 | 100 | 100 |
| NMPF-43 | 100 | 100 | 100 | 100 |
| NMPF-45 | 100 | 100 | 100 | 100 |
| NMPF-46 | 100 | 100 | 100 | 100 |
| NMPF-50 | 100 | 100 | 100 | 100 |
| NMPF-53 | 100 | 100 | 100 | 100 |
| NMPF-58 | 100 | 100 | 100 | 100 |
| NMPF-60 | 100 | 100 | 100 | 100 |

**TABLE 4. Further additional results**

| NMPF PEPTIDES | | SICKNESS SCORES | | |
|---|---|---|---|---|
| Tx | Tx | | | |
| | 0 | 14 | 24 | 48 |
| | | | | |
| PBS | 0,0,0,0,0,0 | 5,5,5,5,4,4 | 5,5,5,5,5,5 | †††††† |
| NMPF-3 | 0,0,0,0,0,0 | 3,3,3,3,3,4 | 4,4,4,4,4,4 | †††††† |
| NMPF-5 | 0,0,0,0,0,0 | 5,5,5,5,5,5 | 5,5,5,5,5,5 | 2,2,2,2,2,2 |
| NMPF-7 | 0,0,0,0,0,0 | 1,1,4,4,4,4 | 5,5,5,5,5,5 | 2,2,2,2,tt |
| NMPF-8 | 0,0,0,0,0,0 | 3,3,5,5,5,5 | 5,5,5,5,5,5 | 2,2,4,4,4,5 |
| NMPF-9 | 0,0,0,0,0,0 | 3,3,4,4,5,5 | 2,2,2,2,2,2 | 1,1,2,2,2,2 |
| NMPF-11 | 0,0,0,0,0,0 | 1,1,3,3,4,4, | 2,2,2,2,4,4 | 1,1,1,1,1,1 |
| NMPF-12 | 0,0,0,0,0,0 | 1,1,1,1,3,3 | 1,1,1,1,1,1 | 1,1,1,1,1,1 |
| NMPF-43 | 0,0,0,0,0,0 | 1,1,4,4,4,4 | 1,1,1,1,3,3 | 2,2,2,2,2,2 |
| NMPF-45 | 0,0,0,0,0,0 | 5,5,5,5,4,4 | 3,3,4,4,5,5 | 2,2,4,4,5,5 |
| NMPF-46 | 0,0,0,0,0,0 | 1,1,2,2,3,3 | 1,1,2,2,2,2 | 1,1,1,1,1,1 |
| NMPF-50 | 0,0,0,0,0,0 | 1,1,1,1,3,3 | 2,2,2,2,3,3 | 1,1,1,1,1,1 |
| NMPF-53 | 0,0,0,0,0,0 | 5,5,5,5,5,5 | 5,5,5,5,5,5 | 1,1,2,2,2,2 |
| NMPF-58 | 0,0,0,0,0,0 | 5,5,5,5,3,3 | 5,5,5,5,3,3 | 1,1,1,1,1,1 |
| NMPF-60 | 0,0,0,0,0,0 | 1,1,4,4,2,2 | 2,2,2,2,4,4 | 1,1,1,1,1,1 |

**Table 5 Summary of results of the various peptides in the various experiments.**

| **ID** | **SEQUENCE** | **SEPSIS** | **ANGIOGENSIS** | **CAO** | **DC** | **NOD** |
|---|---|---|---|---|---|---|
| **NMPF-1** | VLPALPQVVC | -+ | | + | + | |
| **NMPF-2** | LQGVLPALPQ -+ | | | | + | |
| **NMPF-3** | LOG | -+ | + | + | + | |
| **NMPF-4** | LQGV | + | + | + | + | |
| **NMPF-5** | GVLPALPQ | -+ | | | + | |
| **NMPF-6** | VLPALP | + | + | + | + | |
| **NMPF-7** | VLPALPQ | + | + | | + | |
| **NMPF-6** | GVLPALP | -+ | | | + | |
| **NMPF-9** | VVC | + | + | | + | |
| **NMPF-10** | QVVC | | | | | |
| **NMPF-11** | MTRV | + | + | | + | + |
| **NMPF-12** | MTR | -+ | + | | + | |
| **NMPF-13** | LQGVLPALPQVVC | + | | | + | |
| **NMPF-14** | cyclic- LQGVLPALPQVVC | + | | | | |
| **NMPF-43** | AQG | + | + | | + | |
| **NMPF-44** | LAG | | + | | | |
| **NMPF-45** | LQA | + | + | | | |
| **NMPF-46** | AQGV | + | + | | + | |
| **NMPF-47** | LAGV | -+ | | + | + | |
| **NMPF-48** | LQAV | | | | | |
| **NMPF-49** | LQGA | + | | | | |
| **NMPF-50** | ALPALP | + | | | + | |
| **MMPF-51** | VAPALP | + | + | | | |
| **NMPF-52** | VLAALP | | | | | |
| **NMPF-53** | VLPAAP | + | | | + | |
| **NMPF-54** | VLPALA | | | | | |
| **NMPF-55** | ALPALPQ | + | | | | |
| **NMPF-56** | VAPALPQ | | + | | | |
| **NMPF-57** | VLAALPQ | | | | | |
| **NMPF-58** | VLPAAPQ | + | | | + | |
| **NMPF-59** | VLPALAQ | + | + | | | |
| **NMPF-60** | VLPALPA | + | | | + | |
| **NMPF-61** | VVCNYRDVRFESIRLPGCPRGVNPVVSYAVALSCQCAL | -+ | | + | | |
| **NMPF-62** | VVCNYRDVRFESIRLPGCPRGVNPVVSYAVALSCQ | | | | | |
| **NMPF-63** | SIRLPGCPRGVNPVVS | -+ | | | | |
| **NMPF-64** | LPGCPRGVNPVVS | | | + | | |
| **NMPF-65** | CPRGVNPVVS | | | | | |
| **NMPF-66** | LPGC | + | + | + | | |
| **NMPF-67** | CPRGVNP | | | | | |
| **NMPF-68** | PGCP | -+ | | | | |
| **NMPF-69** | RPRCRPINATLAVEKEGCPVCITVNTTICAGYCPT | | | | | |
| **NMPF-70** | MTRVLQGVLPALPQ | -+ | | | | |
| **NMPF-71** | MTRVLPGVLPALPQVVC | -+ | | | | |
| **NMPF-74** | CALCRRSTTDCGGPKDHPLTC | | | | | |
| **NMPF-75** | SKAPPPSLPSPSRLPGPC | | | | | |
| **NMPF-76** | TCDDPRFQDSSSSKAPPPSLPSPSRLPGPSDTPILPQ | | | | | |

| | | | | | | |
|---|---|---|---|---|---|---|
| + = effects; -+ = variable effect; no entry is no effect or not yet tested when table was assembled | | | | | | |

### MODULATION OF NO AND/OR

**Table 6 TNF-A**

| **ID** | **SEQUENCE** | **TNF-A** | **NO** | **TNF-A and NO** |
|---|---|---|---|---|
| **NMPF-1** | VLPALPQWC | ++ | ++++ | ++++ |
| **NMPF-2** | LQGVLPALPQ | -+ | ++++ | ++++ |
| **NMPF-3** | LQG | + | ++++ | ++++ |
| **NMPF-4** | LQGV | ++++ | ++++ | +++++++ |
| **NMPF-5** | GVLPALPQ | ++++ | ++++ | +++++++ |
| **NMPF-6** | VLPALP | ++++ | ++++ | +++++++ |
| **NMPF-7** | VLPALPQ | ++++ | ++++ | +++++++ |
| **NMPF-8** | GVLPALP | ++++ | ++++ | +++++++ |
| **NMPF-9** | VVC | ++++ | ++++ | +++++++ |
| **NMPF-10** | QVVC | ++++ | +++ | ++++ |
| **NMPF-11** | MTRV | ++++ | ++++ | ++++ |
| **NMPF-12** | MTR | ++++ | ++++ | ++++ |
| **NMPF-13** | LQGVLPALPQVVC | ++ | ++++ | ++++ |
| **NMPF-14** | cyclic- LQGVLPALPQVVC | ++ | ++++ | ++++ |
| **NMPF-43** | AQG | ++++ | ++++ | +++++++ |
| **NMPF-44** | LAG | -+ | ++++ | ++++ |
| **NMPF-45** | LQA | ++++ | ++++ | +++++++ |
| **NMPF-46** | AQGV | ++++ | ++++ | +++++++ |
| **NMPF-47** | LAGV | ++ | ++++ | ++++ |
| **NMPF-48** | LQAV | ++ | ++++ | ++++ |
| **NMPF-49** | LQGA | ++ | ++++ | ++++ |
| **NMPF-50** | ALPALP | ++++ | ++++ | +++++++ |
| **NMPF-51** | VAPALP | + | +++ | ++++ |
| **NMPF-52** | VLAALP | ++ | ++++ | ++++ |
| **NMPF-53** | VLPAAP | ++++ | ++++ | +++++++ |
| **NMPF-54** | VLPALA | + | ++++ | ++++ |
| **NMPF-55** | ALPALPQ | + | ++++ | ++++ |
| **NMPF-56** | VAPALPQ | -+ | ++++ | ++++ |
| **NMPF-57** | VLAALPQ | + | ++++ | ++++ |
| **NMPF-58** | VLPAAPQ | ++++ | ++++ | +++++++ |
| **NMPF-59** | VLPALAQ | ++ | ++++ | ++++ |
| **NMPF-60** | VLPALPA | ++++ | ++++ | +++++++ |
| **NMPF-61** | VVCNYRDVRFESIRLPGCPRGVN PVVSYAVALSCQCAL | -+ | ++++ | ++++ |
| **NMPF-62** | VVCNYRDVRFESIRLPGCPRGVN PVVSYAVALSCQ | -+ | +++ | ++++ |
| **NMPF-63** | SIRLPGCPRGVNPVVS | -+ | ++ | ++ |
| **NMPF-64** | LPGCPRGVNPVVS | ++ | ++++ | ++++ |
| **NMPF-65** | CPRGVNPVVS | ++ | +++ | +++ |
| **NMPF-66** | LPGC | +++ | ++ | +++ |
| **NMPF-67** | CPRGVNP | -+ | + | + |
| **NMPF-68** | PGCP | + | + | +++ |
| **NMPF-69** | RPRCRPINATLAVEK EGCPVCITVNTTICAGYCPT | -+ | ++ | ++ |
| **NMPF-70** | MTRVLQGVLPALPQ | -+ | + | + |
| **NMPF-71** | MTRVLPGVLPALPQVVC | -+ | -+ | -+ |
| **NMPF-74** | CALCRRSTTDCGGPKDHPLTC | -+ | ++ | + |
| **NMPF-75** | SKAPPPSLPSPSRLPGPS | + | ++ | ++ |
| **NMPF-76** | TCDDPRFQDSSSSKAPPPSLPSPS RLPGPSDTPILPQ | + | + | + |
| **NMPF-78** | CRRSTTDCGGPKDHPLTC | + | + | + |

| | | | | |
|---|---|---|---|---|
| from -+ to +++++++ indicates from barely active to very active in modulating | | | | |

## Claims

1. An isolated peptide consisting of the amino acid sequence AQG.

2. An isolated peptide consisting of the amino acid sequence AQG for use as medicament.

3. A pharmaceutical preparation comprising as an active component an isolated peptide consisting of the amino acid sequence AQG.

4. An isolated peptide consisting of the amino acid sequence AQG for the treatment of sepsis.

5. An isolated peptide consisting of the amino acid sequence AQG for the inhibition of angiogenesis.

## Patentansprüche

1. Isoliertes Peptid, das aus der Aminosäuresequenz AQG besteht.

2. Isoliertes Peptid, das aus der Aminosäuresequenz AQG besteht, zur Verwendung als Medikament.

3. Pharmazeutische Zusammensetzung, die als aktiven Bestandteil ein isoliertes Peptid umfasst, welches aus der Aminosäuresequenz AQG besteht.

4. Isoliertes Peptid, das aus der Aminosäuresequenz AQG besteht, zur Behandlung der Sepsis.

5. Isoliertes Peptid, das aus der Aminosäuresequenz AQG besteht, zur Inhibierung der Angiogenese.

## Revendications

1. Peptide isolé consistant en la séquence d'aminoacides AQG.

2. Peptide isolé consistant en la séquence d'aminoacides AQG destiné à être utilisé comme médicament.

3. Préparation pharmaceutique comprenant comme composant actif un peptide isolé consistant en la séquence d'aminoacides AQG.

4. Peptide isolé consistant en la séquence d'aminoacides AQG pour le traitement de la septicémie.

5. Peptide isolé consistant en la séquence d'aminoacides AQG pour l'inhibition de l'angiogenèse.
